# EUROPEAN PATENT APPLICATION

(11) **EP 4 609 807 A1**
(43) Date of publication of application: **03.09.2025**
(21) Application number: 25161023.4
(22) Date of filing: 28.02.2025
(51) Int. Cl.: A61B 17/70

(54) **SCREW INSERTER**

(30) Priority: 01.03.2024 US 202463560251 P
(71) Applicant: Stryker Corporation, Portage, MI 49002 (US)
(72) Inventor: MCCLINTOCK, Larry, GORE, 22637 (US); MOORE, Jennifer Anne, LEESBURG, 20175 (US); MEYER, Joseph Michael, TERRACE PARK, 45174 (US); BUCHERT, Oliver, FRANKLIN LAKES, 07417 (US); FREDERICKS, Jospeh, DUMONT, 07628 (US); HAILEYESUS, Ailon, DUMFRIES, 22026 (US)
(74) Representative: Regimbeau

(57) **Abstract**

A screw inserter for driving a screw into a vertebral body includes an inner element for engaging a screw portion of the screw and an outer element for engaging a coupling element of the screw. The outer element may include a threaded distal end. In addition, the screw inserter may include a button permitting the translation of the inner element with respect to the outer element. Further, the inserter may include an outer knob allowing the rotation of the inner element with respect to the outer element when moved to a first position. When the outer knob is moved to a second position it prevents the rotation of the inner element with respect to the outer element.

## Description

### CROSS-REFERENCE TO RELATED APPLICATIONS

This application claims the benefit of the filing date of United States Provisional Patent Application No. 63/560,251 filed on March 1, 2024, the disclosure of which is hereby incorporated by reference.

### BACKGROUND OF THE INVENTION

Spinal screws, such as pedicle screws and lateral mass screws, have been used for many years by surgeons to correct degenerative spine diseases, spinal column injuries, and spinal column deformities. A typical screw procedure involves the implantation of screws into the different portions of different level vertebral bodies and their connection via a spinal rod or the like. This may or may not be done in concert with the implantation of an intervertebral implant, with the goal being the fusion of the vertebral bodies to each other.

Implantation of the screws into the vertebral bodies typically involves the use of a screw inserter to drive the screws into the vertebral bodies. These inserters may include inner and outer elements, with the inner element being designed to engage and drive the screw portion into the vertebral body, while the outer element attaches to the tulip or coupling element of the screw. Although these types of inserters generally operate for their intended purpose, their outer elements can catch on soft tissue during screw insertion, causing it to rotate and unthread from the screw. As a result, the screw is prone to accidentally uncoupling from the inserter during screw insertion.

Screw inserters have been designed to permit the locking of inner and outer elements to address the foregoing issues. While these involve different mechanisms to achieve the locking, they typically result in overly complicated and bulky designs. Other drawbacks may also include inner element tips that are susceptible to breaking due to uneven torque loading and actuation mechanisms that are susceptible to being actuated by mistake. These drawbacks can have particularly negative effects in cervical spine procedures, which tend to be more delicate than thoracic and lumbar procedures.

Thus, there is a need for a user-friendly screw inserter with a streamlined locking procedure that eliminates the known drawbacks.

### BRIEF SUMMARY OF THE INVENTION

The present invention addresses the aforementioned drawbacks by providing a simple to use screw inserter that can lock inner and outer elements of the inserter both rotationally and translationally. The inserter includes one actuation mechanism for preventing/permitting rotational movement of the inner and outer elements with respect to each other and one for prevent/permitting translational movement. These are configured to provide a clear and simple operation for a user.

In accordance with an aspect of the present disclosure, a tool for driving a screw (*e.g.,* a pedicle or lateral mass screw) into a vertebral body includes an inner element, including a distal end for engaging a screw portion of the screw, an outer element, including a threaded distal end for engaging a coupling element of the screw, a button, configured to permit translation of the inner element with respect to the outer element, and an outer knob. Movement of the outer knob to a first position permits rotation of the inner element with respect to the outer element and movement of the outer knob to a second position prevents rotation of the inner element with respect to the outer element. The tool also includes a locking button housed in an inner knob on the inner element.

In another aspect, the tool includes a ramped surface on the inner element configured to enable passive translation of the inner element with respect to the outer element.

In a different aspect, the inner element includes a more proximal reduced diameter and a more distal reduced diameter.

In a further aspect, the more proximal reduced diameter is configured to translate the inner element distally and the more distal reduced diameter is configured to facilitate passive disengagement of the threaded distal end from the screw.

In another aspect, the button includes a spring-loaded button.

In a different aspect, the tool includes an inner knob on the inner element including the button and a locking button.

In a further aspect, the tool includes an angled slot on the outer knob configured to attach to the inner knob and allow axial translation from the first position to the second position.

In another aspect, the locking button is configured to translate inward via an interface between the ramped surface and the outer knob, when the outer knob is moved distally into the second position.

In a different aspect, the tool includes a cut spline on the inner element that engages with the locking button in the second position.

In a further aspect, the outer knob translates proximally to disengage the locking button and rotationally decouple the inner element from the outer element.

In another aspect, the locking button includes a pawl configured to engage the cut spline on the inner element to prevent rotation in one or both directions.

In a different aspect, the pawl includes a spring-loaded pawl rotatably constrained to the locking button and configured to engage the cut spline when the locking button is depressed.

In a further aspect, the locking button includes a stop face above and below the spring-loaded pawl configured to prevent the spring-loaded pawl from moving further than intended.

In another aspect, the locking button includes a first pawl configured to translate and engage the cut spline, preventing rotation in one direction.

In yet another aspect, the locking button includes a second pawl that mirrors the first pawl about a vertical axis to the cut spline, preventing rotation in both directions.

In yet another aspect, the locking button includes a cantilever beam flexure configured to engage the cut spline and prevent rotation in one direction.

In yet another aspect, the locking button includes a cartwheel flexure, with a pivot point at an intersection of two connected flexures, configured to engage the cut spline and prevent rotation in one direction.

In yet another aspect, the locking button includes a small-length flexural pivot configured to engage the cut spline and prevent rotation in one direction.

In yet another aspect, the locking button includes a cross-axis flexural pivot, with a pivot point at an intersection of two disconnected flexures, configured to engage the cut spline and prevent rotation in one direction.

In yet another aspect, the locking button includes a beam flexure with teeth configured to engage the cut spline and prevent rotation in one or both directions.

In a further aspect, the locking button includes the beam flexure with at least one slot cut into the side of the beam flexure to create multiple flexures with a reduced width and stiffness, configured to engage the cut spline and prevent rotation in one direction.

In a different aspect, the locking button includes a set of left and right-side beam flexures, having at least one slot cut into the side of the beam flexure, that are intended to flex independently from each other and are configured to engage the cut spline and prevent rotation in one or both directions.

In accordance with yet another aspect of the present disclosure, a method of inserting a screw into a vertebral body includes threading an outer element of an inserter to a coupling element of a screw, engaging a distal end of an inner element of the inserter to a screw portion of the screw, translating an outer knob of the inserter distally to rotationally lock the inner element with respect to the outer element, driving the screw into a vertebral body via the inserter, translating the outer knob of the inserter proximally to rotationally unlock the inner element from the outer element, and unthreading the outer element from the coupling element.

### BRIEF DESCRIPTION OF THE DRAWINGS

A more complete appreciation of the subject matter of the present invention and of the various advantages thereof can be realized by reference to the following detailed description in which reference is made to the accompanying drawings in which:
FIG. 1 is a perspective view of a locking screw inserter assembly in an unlocked and retracted position.
FIG. 2 is a perspective view of the locking screw inserter assembly of FIG. 1 in a locked and retracted position.
FIG. 3 is a perspective view of the locking screw inserter assembly of FIG. 1 in an unlocked and extended position.
FIG. 4 is an elevation view of an outer knob of the locking screw inserter assembly of FIG. 1 in an unlocked position.
FIG. 5 is an elevation view of the outer knob of the locking screw inserter assembly of FIG. 1 in a locked position.
FIG. 6 is a perspective view of an inner element of the locking screw inserter assembly of FIG. 1.
FIG. 7 is a perspective view of the locking screw inserter assembly according to a different embodiment in a locked position, with certain portions thereof hidden.
FIG. 8 is an enlarged perspective view of the locking screw inserter assembly of FIG. 7 focusing on an inner knob.
FIG. 9 is a perspective view of the locking screw inserter assembly of FIG. 1 in an unlocked position, with certain portions thereof hidden.
FIG. 10 is an enlarged perspective view of the locking screw inserter assembly of FIG. 9 focusing on the inner knob.
FIG. 11 is a partial cross-sectional view focusing on a button and a locking button inside the inner knob according to an embodiment of the present invention.
FIG. 12 is a partial cross-sectional view focusing on the button and the locking button inside the inner knob, with a different inner element embodiment according to the present invention.
FIG. 13 is an elevation view of the button according to an embodiment of the present invention.
FIG. 14 is an enlarged elevation view of the locking button according to an embodiment of the present invention.
FIG. 15 is an elevation view of the locking button according to an embodiment of the present invention.
FIG. 16 is an elevation view of the locking button according to an embodiment of the present invention.
FIG. 17 is an elevation view of the locking button according to an embodiment of the present invention.
FIG. 18 is an enlarged elevation view of the locking button according to an embodiment of the present invention.
FIG. 19 is an elevation view of the locking button according to an embodiment of the present invention.
FIG. 20 is an elevation view of the locking button according to an embodiment of the present invention.
FIG. 21 is an elevation view of the locking button according to an embodiment of the present invention.
FIG. 22 is a perspective view of the locking button according to an embodiment of the present invention.
FIG. 23 is a perspective view of the locking button according to an embodiment of the present invention.
FIG. 24 is a perspective view of the locking button according to an embodiment of the present invention.
FIG. 25 is an enlarged perspective view of a portion of the locking button according to an embodiment of the present invention.
FIG. 26 is an enlarged perspective view of a portion of the locking button according to an embodiment of the present invention.
FIG. 27 is an enlarged perspective view of a portion of the locking button according to an embodiment of the present invention.
FIG. 28 is a perspective view of the locking button according to an embodiment of the present invention.
FIG. 29 is a perspective view of the locking button according to an embodiment of the present invention.
FIG. 30 is an elevation view of the locking button according to an embodiment of the present invention.
FIG. 31 is a perspective view of the locking button of FIG. 30.
FIG. 32 is a partial cross-sectional view focusing on the locking button of FIG. 30 inside the inner knob.

### DETAILED DESCRIPTION

In describing the preferred embodiments of the invention, specific terminology will be used for the sake of clarity. However, the invention is not intended to be limited to any specific terms used herein, and it is to be understood that each specific term includes all technical equivalents, which operate in a similar manner to accomplish a similar purpose. In the drawings and in the description which follows, the term "proximal" refers to the end of the instrument, or portion thereof, which is closest to the operator in use, while the term "distal" refers to the end of the instrument, or portion thereof, which is farthest from the operator in use.

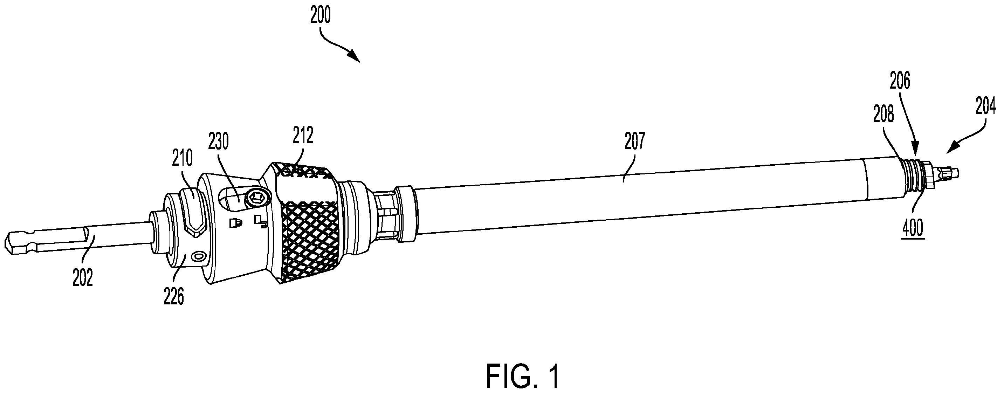

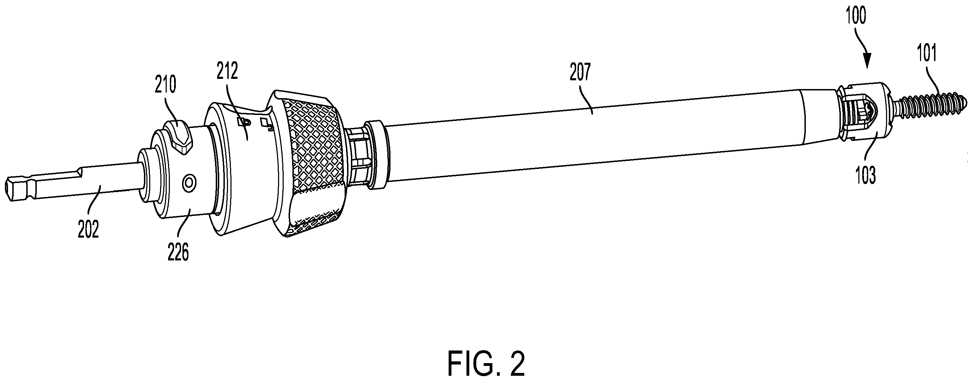

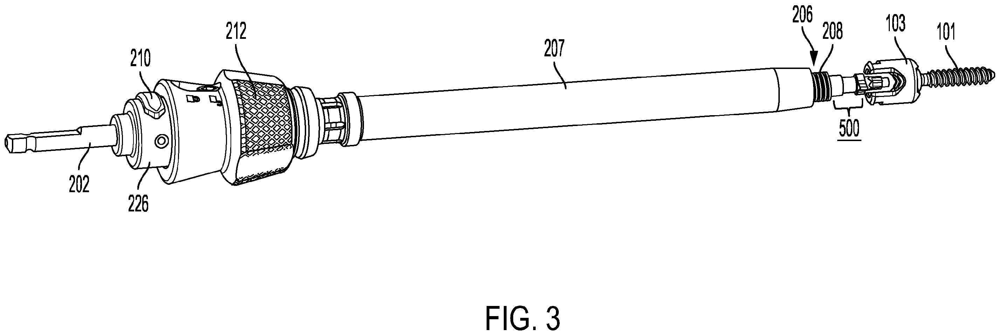

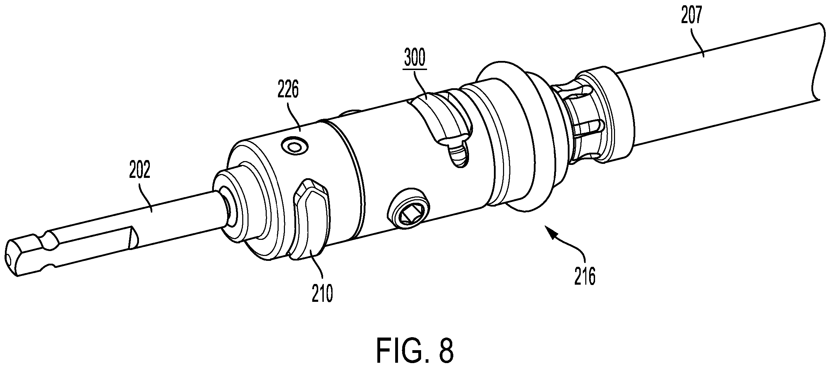

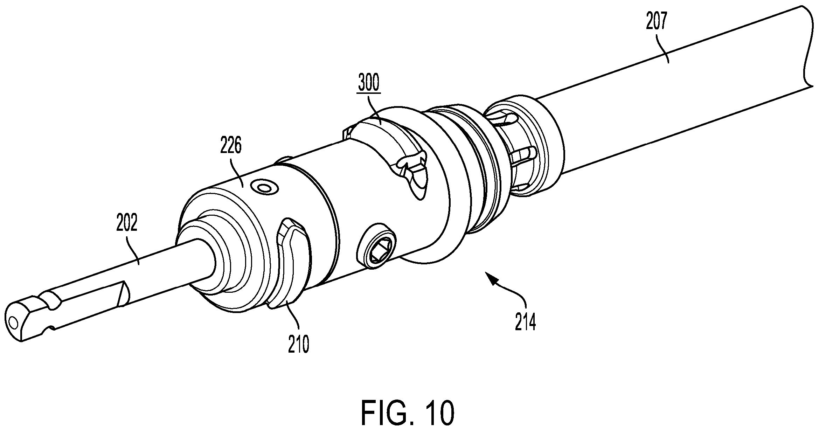

Referring to FIGS. 1-3, a screw inserter ("inserter") 200 according to an embodiment of the present invention includes an inner element 202, an outer element 206, a button 210 and an outer knob 212. Outer element 206 is incased in an outer case 207. As better shown in FIGS. 8 and 10-12, inserter 200 may also include an inner knob 226 with a locking button 300. With particular reference to FIGS. 2 and 3, inner element 202 includes a distal end 204 for engaging a screw portion 101 of a screw 100 (*e.g.,* pedicle screw or lateral mass screw) and outer element 206 includes a threaded distal end 208 for engaging a coupling element 103 of screw 100. Button 210 is housed within inner knob 226 and a portion of inner element 202 is designed to extend through the button. As will be described in more detail below, depressing button 210 permits translation of inner element 202 with respect to outer element 206 of inserter 200 from a retracted position 400 into an extended position 500.

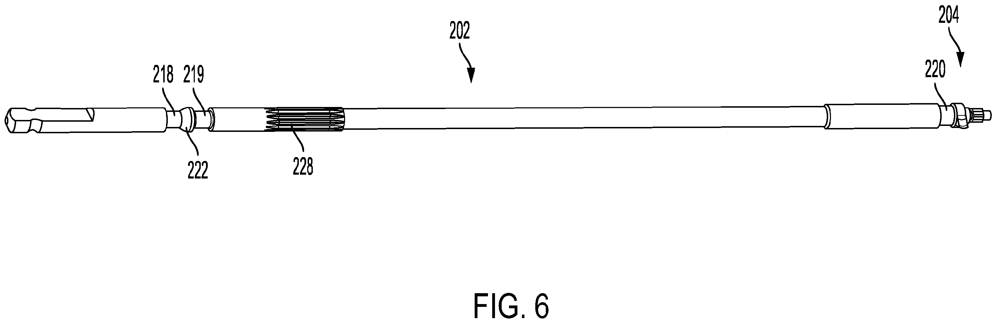

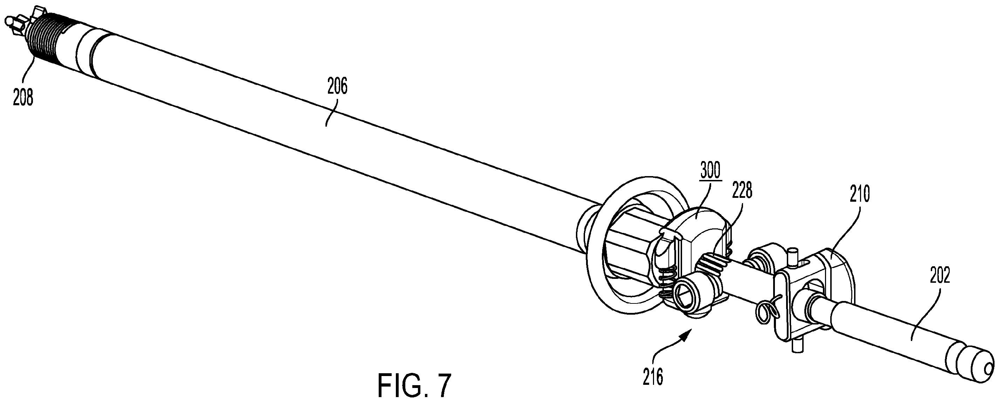

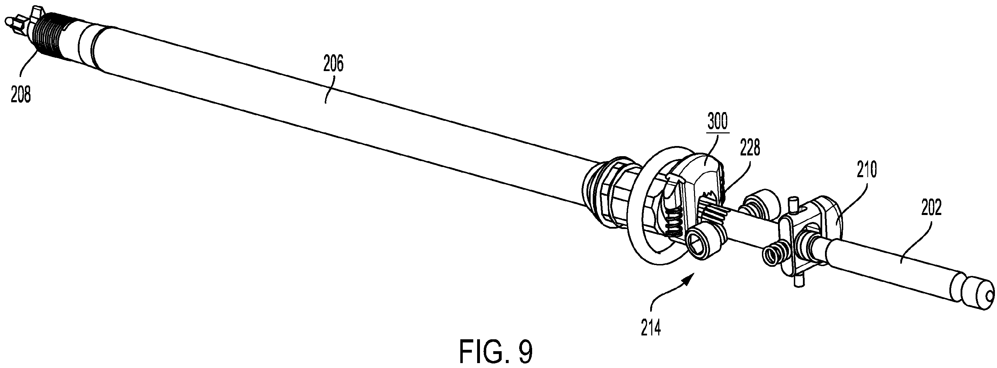

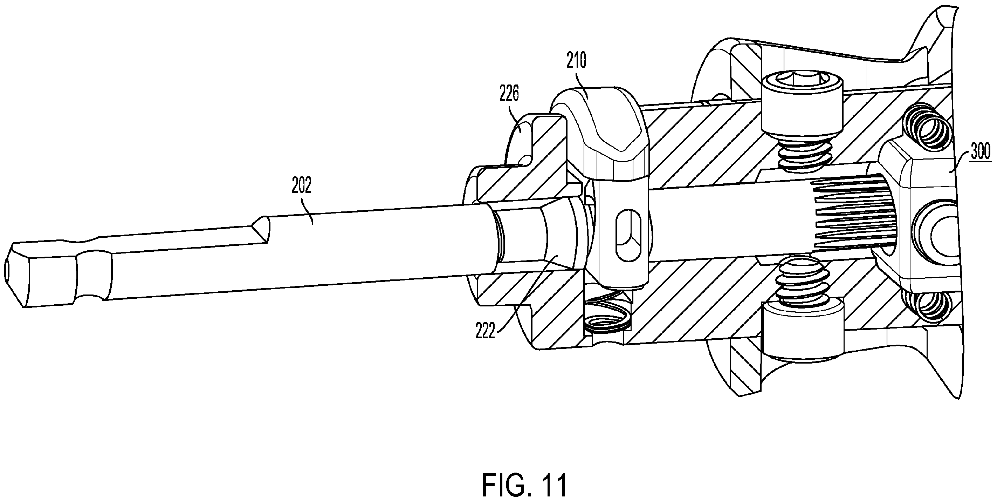

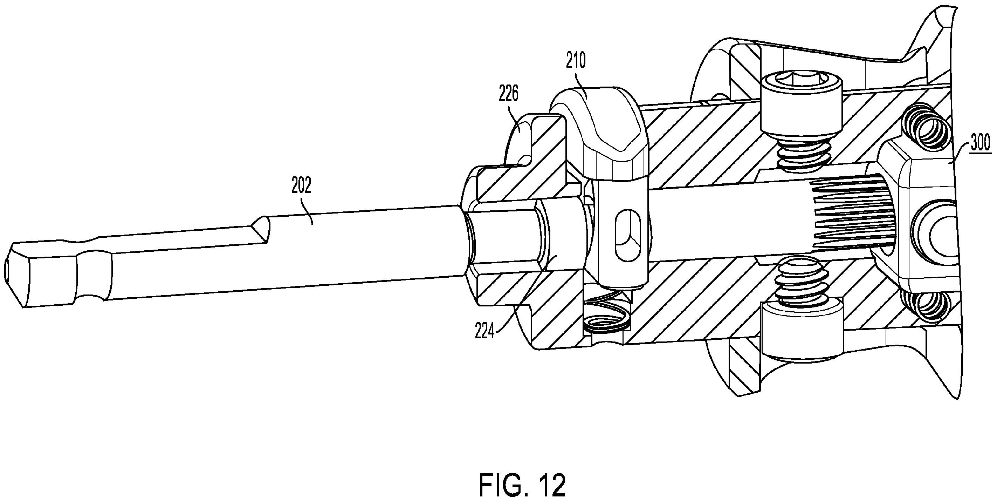

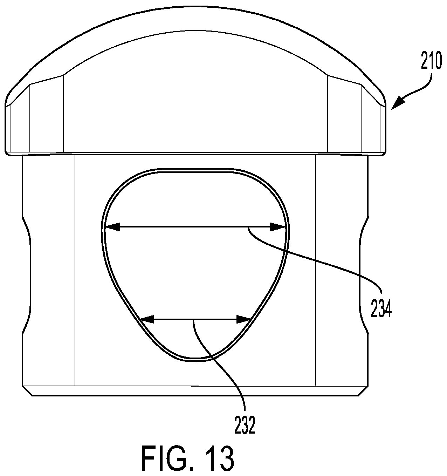

As shown in FIG. 6, in addition to distal end 204, inner element 202 includes three reduced diameters - a first more proximal reduced diameter 218, a second more proximal reduced diameter 219, and a more distal reduced diameter 220. First more proximal reduced diameter 218 and second more proximal reduced diameter 219 are separated by ramped surface 222 and are configured to lock inner element 202 in two different translational positions. As shown in FIG. 13, button 210 includes a first inner width 232 that is less than the diameter of inner element 202, which allows inner element to remain in retracted position 400 with button contacting second more proximal reduced diameter 219. Depressing button 210 allows inner element 202 to shift to a second inner width 234 of button, that is greater than the diameter of inner element, which allows inner element to translate into extended position 500 with first more proximal reduced diameter 218 contacting button. As shown in FIGS. 7 and 9, inner element 202 can only translate into these positions when button 210 is depressed, and reduced diameters 218, 219 move into second inner width 234 of button. As shown in FIGS. 11 and 12, in one embodiment of the present invention, button 210 is biased to the position in which inner element 202 engages width 232 via a spring 203.

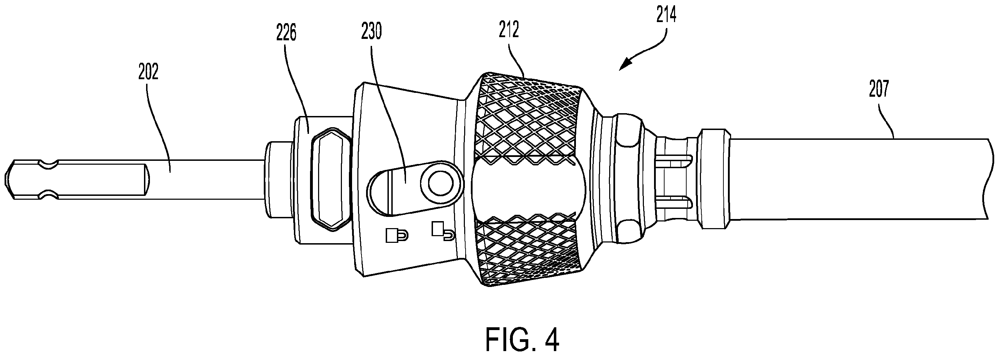

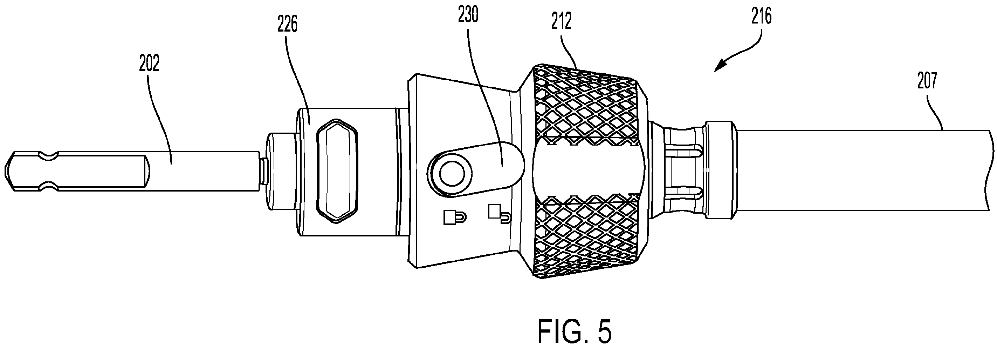

As shown in FIGS. 4-5 and 7-10, movement of outer knob 212 to first position 214 (FIG. 4) permits rotation of inner element 202 with respect to outer element 206, *i.e.,* an unlocked position of inserter 200. Moving outer knob 212 into second position 216 (FIG. 5) prevents rotation of inner element 202 with respect to outer element 206, *i.e.,* a locked inserter 200. Outer knob 212 includes angled slot 230 configured to permit extension of inner knob 226 to slide therein and allow axial translation from first position 214 to second position 216.

In addition to button 210, inner knob 226 also includes locking button 300, which is configured to be depressed or translated inward via an interface between a ramped inner surface of the outer knob and outer knob 212 when outer knob is moved distally into second position 216. When locking button 300 is depressed, a portion of the locking button engages with a cut spline 228 on inner element 202 in second position 216. This rotationally locks the inner and outer elements to each other. To unlock inserter 200, outer knob 212 translates proximally back to first position 214 to disengage locking button 300 and decouple inner element 202 from outer element 206 rotationally.

Locking buttons according to the present invention may exhibit various configurations, most notable in connection with the manner in which the button engages with a cut spline. Reference will now be made to various embodiment splines and locking buttons. Wherever possible, the same or like reference numbers will be used throughout the drawings to refer to the same or like features within a different 100-series of numbers.

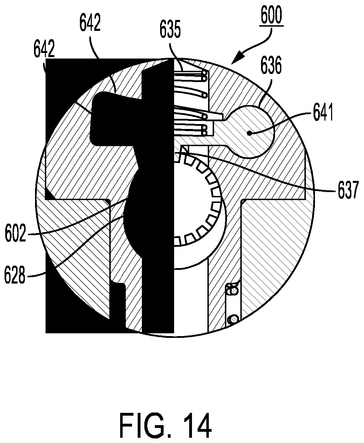

Beginning with FIG. 14, locking button 600 and cut spline 628 are shown. Locking button 600 includes a spring-loaded pawl 636 with a tooth 637 that engages cut spline 628 to prevent rotation of inner element 602 in both rotational directions. Locking button 600 includes stop faces 642 that constrain spring-loaded pawl 636, which is rotatably attached to locking button 600 at pivot point 641. A spring 635 biases pawl 636 such that engagement of tooth 637 with spline 628 is ensured. Locking button springs (not shown) allow locking button 600 to depress to engage cut spline 628 when outer knob (not shown) is translated distally into second position (not shown).

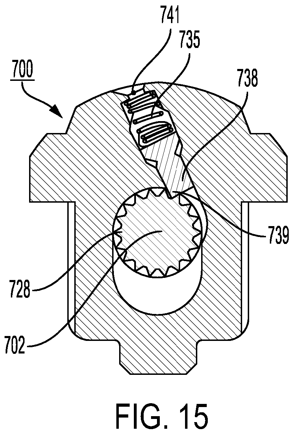

As shown in FIG. 15, another embodiment of the present invention includes locking button 700 comprising a first pawl 738 configured to translate and engage cut spline 728 to prevent rotation. In particular, first pawl 738 includes an edge 739 that engages cut spline 728 to prevent rotation of inner element 702 in at least one direction. Spring 735 biases first pawl 738 such that engagement of edge 739 with cut spline 728 is ensured when locking button 700 is depressed and cut spline moves upward into contact with first pawl. The moveability of first pawl 738 permits engagement even when cut spline 728 is not in a specific orientation with respect to first pawl, as well as permitting rotation of inner element 702 in one direction. Locking button 700 depresses in a similar fashion to locking button 600 discussed above.

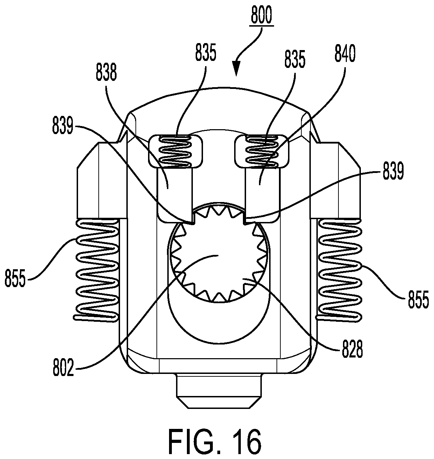

FIG. 16 depicts another embodiment locking button 800 that includes first and second pawls 838, 840 that are spaced apart from each other, but which can both engage spline 828. First pawl 838 and second pawl 840 are configured to translate vertically and engage cut spline 828, much like in connection with pawl 738. However, the inclusion of two pawls in this embodiment prevents rotation in both directions. First pawl 838 and second pawl 840 each include edges 839 that engage cut spline 828 at the same time when locking button 800 is depressed via locking button springs 855 to prevent rotation in both directions. In particular, locking button 800 includes first pawl 838 and second pawl 840 attached to locking button 800 via springs 835 that bias first pawl 838 and second pawl 840 such that engagement of edge 839 with cut spline 828 is ensured via vertical translation when first and second pawls are moved towards cut spline. Locking button 800 depresses in a similar fashion to locking button 600 discussed above.

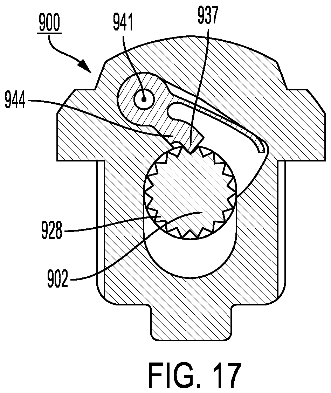

As shown in FIG. 17, another embodiment locking button 900 includes cantilever beam flexure 944. Cantilever beam flexure 944 engages cut spline 928 on inner element 902 to prevent rotation in one direction when locking button 900 is depressed. In particular, locking button 900 includes cantilever beam flexure 944 having tooth 937 configured to engage cut spline 928 to prevent rotation in one direction. Cantilever beam flexure 944 is rotatably attached to locking button 900 at pivot point 941 configured to contact cut spline 928 with tooth 937 when locking button is depressed and cut spline moves upward. The design of cantilever beam flexure 944 is such that tooth 937 is biased towards spline 928 without the need for a separate spring element. Locking button 900 depresses in a similar fashion to locking button 600 discussed above.

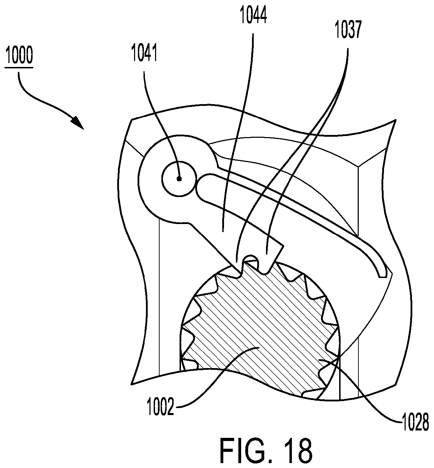

Another embodiment locking button 1000 is shown in FIG. 18 and includes a similar embodiment cantilever beam flexure 1044. Cantilever beam flexure 1044 engages cut spline 1028 on inner element 1002 to prevent rotation in both directions when locking button 1000 is depressed. In particular, cantilever beam flexure 1044 includes teeth 1037 configured to engage cut spline 1028 to prevent rotation in both directions. Cantilever beam flexure 1044 otherwise operates in a similar fashion to flexure 944 discussed above. Locking button 1000 depresses in a similar fashion to locking button 600 discussed above.

In other embodiments, locking button may include a one-piece construction including an integral portion that engages the cut spline to prevent rotation in one or both directions. Such locking buttons depress in a similar fashion to locking button 600 discussed above via locking button springs. The remaining embodiments shown in the figures exhibit such a design.

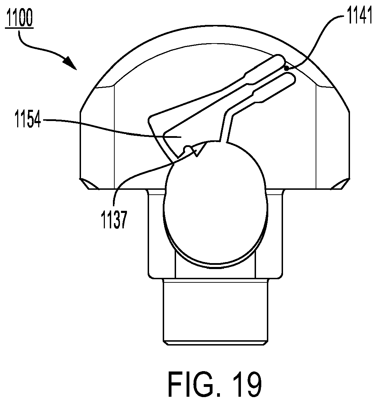

As shown in FIG. 19, another embodiment of locking button 1100 includes a one-piece construction with a small-length flexural pivot 1154. Small-length flexural pivot 1154 engages cut spline (not shown) preventing rotation in one direction when locking button 1100 is depressed. In particular, small-length flexural pivot 1154 includes tooth 1137 configured to engage cut spline to prevent rotation in one direction. Small-length flexural pivot 1154 is rotatably attached to locking button 1100 at pivot or flex point 1141. Small-length flexural pivot 1154 is made of a flexible material allowing for bending as locking button 1100 is depressed to engage cut spline.

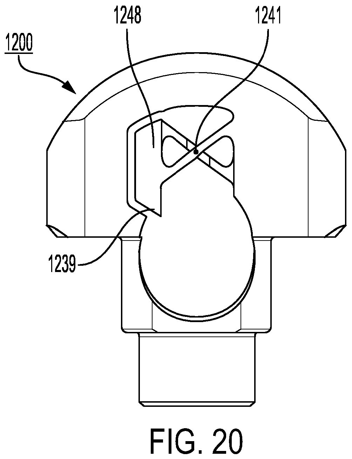

As shown in FIG. 20, another embodiment of locking button 1200 includes a one-piece construction with a cross-axis flexural pivot 1248, that has pivot or flex point 1241 at an intersection of two disconnected flexures. Cross-axis flexural pivot 1248 engages cut spline (not shown) and prevents rotation in one direction when locking button 1200 is depressed. In particular, cross-axis flexural pivot 1248 includes edge 1239 configured to engage cut spline to prevent rotation in one direction. Cross-axis flexural pivot 1248 is made of a flexible material allowing for bending as locking button 1200 is depressed to engage cut spline.

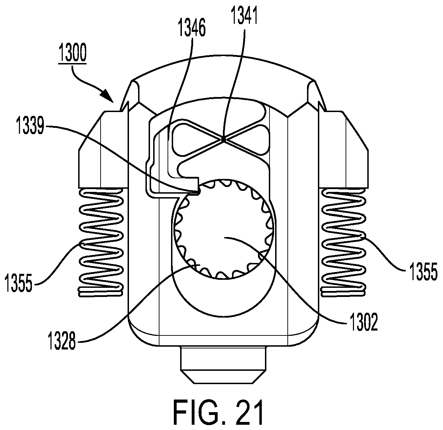

As shown in FIG. 21, another embodiment of locking button 1300 includes a one-piece construction with a cartwheel flexure 1346, that has pivot or flex point 1341 at an intersection of two connected flexures. Cartwheel flexure 1346 engages cut spline 1328, preventing rotation in one direction when locking button 1300 is depressed. In particular, cartwheel flexure 1346 includes edge 1339 configured to engage cut spline 1328 to prevent rotation in one direction. Cartwheel flexure 1346 is made out of a flexible material allowing flexure to bend and rotate around pivot point 1341 to engage cut spline 1328 as locking button 1300 is depressed via locking button springs 1355.

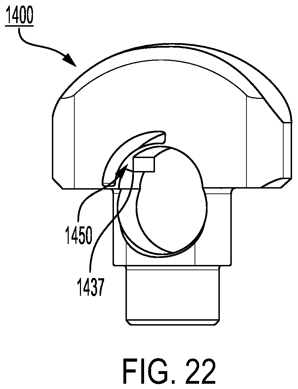

As shown in FIG. 22, another embodiment of locking button 1400 includes beam flexure 1450 with tooth 1437 that engages cut spline (not shown) when locking button 1400 is depressed to prevent rotation in both directions. Beam flexure 1450 is made out of a flexible material allowing beam flexure to bend for easier engagement of tooth 1437 with cut spline as locking button 1400 is depressed.

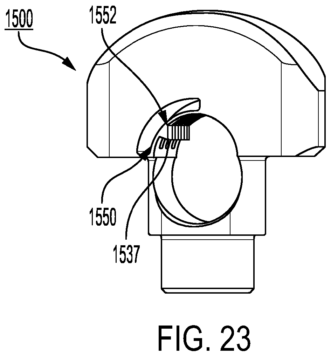

As shown in FIG. 23, another embodiment of locking button 1500 includes beam flexure 1550 with tooth 1537 having at least one slot 1552 cut into the side of beam flexure. Slots 1552 create multiple flexures with a reduced width and stiffness intended to increase the flexibility of beam flexure 1550 when engaging the cut spline (not shown). When locking button 1500 is depressed, tooth 1537 will engage the cut spline. Exact alignment between tooth 1537 and cut spline is not required to prevent rotation because beam flexure 1550 may be made of flexible material allowing for easier engagement of tooth by cut spline.

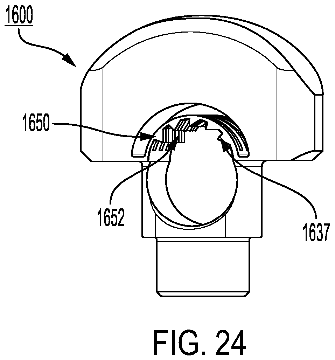

As shown in FIG. 24, another embodiment of locking button 1600 includes beam flexure 1650 with teeth 1637 having at least one slot 1652 cut into the side of beam flexure. Slots 1652 create multiple flexures with a reduced width and stiffness intended to increase the flexibility of beam flexure 1650 when engaging cut spline (not shown). This design is similar to that of FIG. 23, albeit with more teeth for engaging the cut spline.

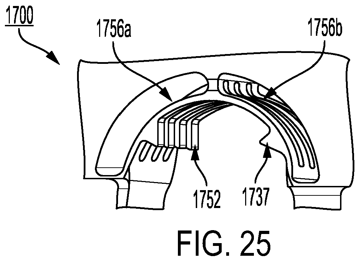

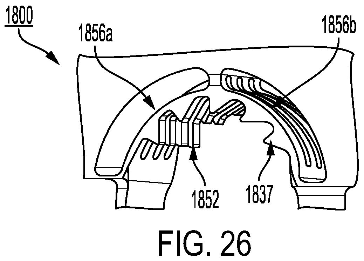

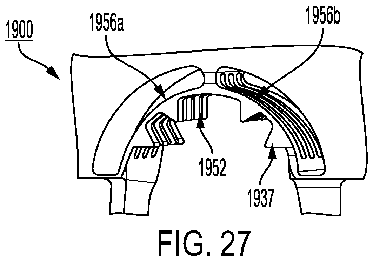

Additional embodiment of locking buttons 1700, 1800, 1900 are shown in FIGS. 25-27. These each include a set of left and right-side beam flexures 1756a, 1756b; 1856a, 1856b; 1956a, 1956b having at least one slot 1752, 1852, 1952 cut into the side of each beam. Each beam flexure 1756a, 1756b; 1856a, 1856b; 1956a, 1956b is intended to flex independently from each other to engage cut spline (not shown), preventing rotation in one or both directions. In particular, beam flexures 1756a, 1756b; 1856a, 1856b; 1956a, 1956b include teeth 1737, 1837, 1937 that are configured to engage cut spline when locking button 1700,1800, 1900 is depressed. The shape of teeth 1737, 1837, 1937 in locking button 1700, 1800, 1900 may vary to prevent rotation in both directions. Exact alignment between teeth 1737, 1837, 1937 and cut spline is not required to prevent rotation because beam flexures 1756a, 1756b; 1856a, 1856b; 1956a,1956b are made of flexible material allowing for easier engagement of teeth by cut spline.

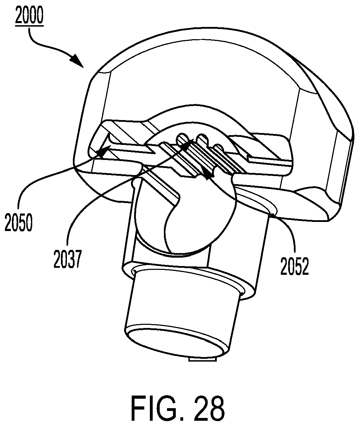

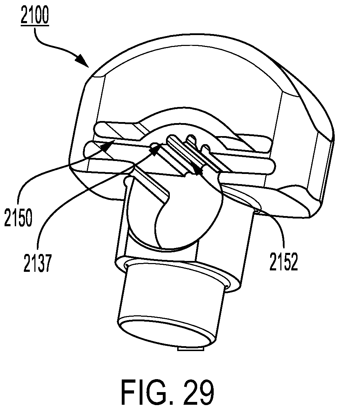

As shown in FIGS. 28 and 29, further embodiment locking button 2000 2100 include a one-piece construction with beam flexures 2050, 2150 including teeth 2037, 2137 configured to flex in order for teeth to meet peak-to-peak with cut spline (not shown) to provide a seamless lock. The in-plane width of beam flexure 2050, 2150 may be tailored to achieve the desired level of flexibility. In some embodiments, locking button 2000, 2100 is made of a flexible material that allows beam flexures 2050, 2150 to bend as locking button is depressed and teeth 2037, 2137 engage cut spline.

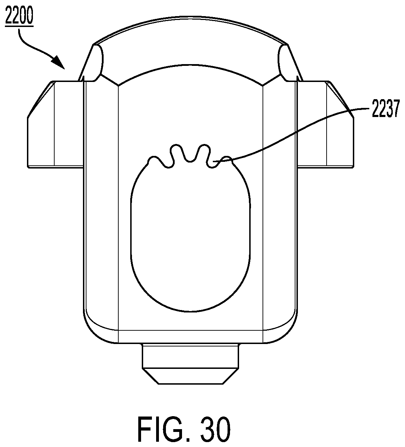

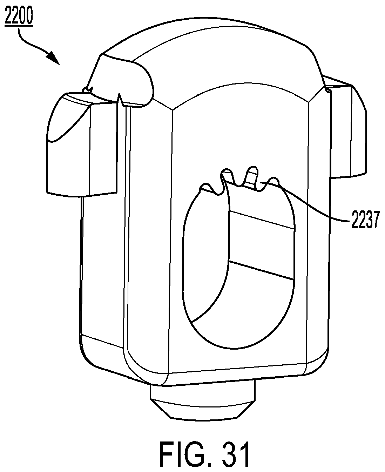

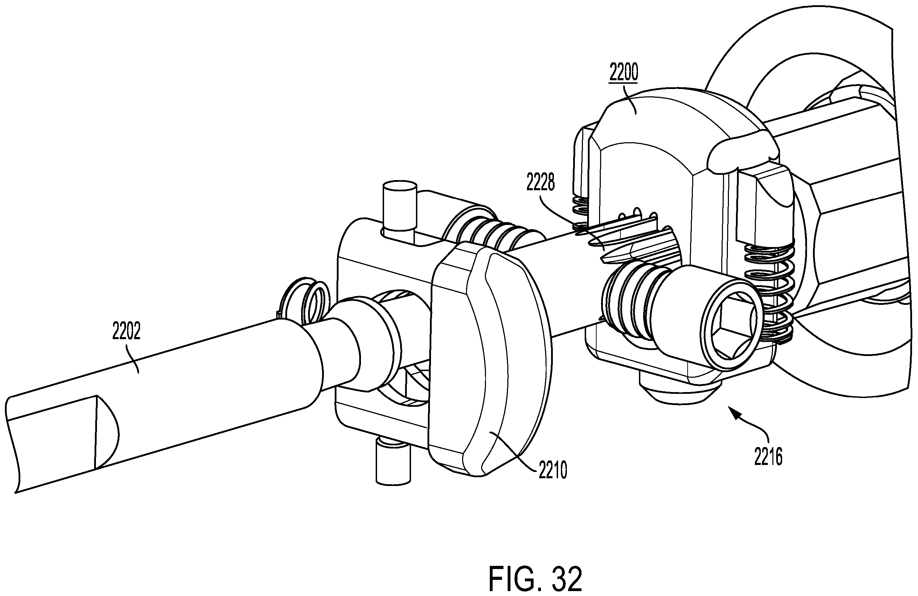

As shown in FIGS. 30-32, another embodiment locking button 2200 also includes a one-piece construction having an opening with teeth 2237 configured to meet peak-to-peak with cut spline 2228. Cut spine 2228 includes curved protrusions configured to fit seamlessly between teeth 2237 of locking button 2200. This provides a seamless lock upon depression of locking button. As shown in FIGS. 30 and 31, locking button 2200 includes three curved teeth 2237 arranged along an upper end of the opening. Such a curvature of teeth 2237 and cut spine 2228 may prevent rotation in one or both directions.

In accordance with another aspect of the present disclosure, a method of inserting screw into vertebral body pedicle includes threading screw 100 onto inserter 200 by mating coupling element of screw 103 with threaded distal end of inserter 208, locking inserter by translating outer knob 214 distally, driving screw into a vertebral body, unlocking inserter by translating outer knob proximally, and unthreading inserter from screw.

As shown in FIG. 3, extended position 500 of inner element 202 facilitates threaded distal end 208 in threading coupling element 103 of screw 100 by engaging coupling element and then proximally pushing screw until threaded distal end contacts coupling element. After extended position 500 engages coupling element 103 of screw 100, according to an embodiment of the present invention with a straight surface 224 on inner element 202, button 210 is depressed to disengage coupling element 103 of screw 100 and enable inner element to retract into inserter 200. Depressing button 210 advances inserter 200 distally and threads inserter into coupling element 103 of screw 100. When inserter 200 is fully threaded into coupling element 103 of screw 100, button 210 will move outward and engage a flat faced flange of the more distal reduced diameter 218 which facilitates disengagement of threaded distal end 208 of inner element 202 from screw 100 during removal of inserter 200. After inserter 200 is fully engaged with screw 100, outer knob 212 is translated distally to put inserter into a second position 216, rotationally locking the inserter. After driving screw portion 101 of screw 100 into a vertebral body, outer knob 212 is translated proximally into a first position 214 to rotationally unlock the inserter 200. Then inserter 200 is unthreaded from the fully seated screw 100 and inner element 202 is pulled out of engagement with coupling element 103 of screw.

According to another embodiment of the present invention, inner element 202 includes a ramped surface 222 that enables passive translation of inner element with respect to the outer element 206 when button 210 is depressed. Ramped surface 222 allows inner element 202 to retract into inserter 200 while pushing screw 100 onto inserter allowing a passive transition from extended position 500 to retracted position 400. Extended position 500 allows screw 100 to be proximally pushed to threaded distal end 208 of inserter 200, and inner element 202 returns to retracted position 400.

It is to be understood that a proximal end of inner element 202 is to be attached to a separate driving element for use during screw insertion into the vertebral body. Indeed, the proximal end is shown having a typical structure for attachment to such devices. For instance, a torque handle and/or surgical drill/driver could be coupled with this structure and utilized during a surgical procedure involving the driver. It is contemplated that other driving devices could utilized and that the proximal end of inner element 202 could employ any connection structure suitable for such use.

Although the invention herein has been described with reference to particular embodiments, it is to be understood that these embodiments are merely illustrative of the principles and applications of the present invention. For example, features described in relation to one particular embodiment may be combined with features of other embodiments described herein. It is therefore to be understood that numerous modifications may be made to the illustrative embodiments and that other arrangements may be devised without departing from the spirit and scope of the present invention as defined in the appended claims.

## Claims

1. A tool (200) for driving a screw (100) into a vertebral body, comprising:
an inner element (202) including a distal end (204) for engaging a screw portion (101) of the screw (100);
an outer element (206) including a threaded distal end (208) for engaging a coupling element (103) of the screw (100);
a button (210), wherein depressing the button (210) permits translation of the inner element (202) with respect to the outer element (206); and
an outer knob (212), wherein movement of the outer knob (212) to a first position (214) permits rotation of the inner element (202) with respect to the outer element (206) and movement of the outer knob (212) to a second position (216) prevents rotation of the inner element (202) with respect to the outer element (206).

2. The tool of claim 1, further comprising a ramped surface (222) on the inner element (202) configured to enable passive translation of the inner element (202) with respect to the outer element (206).

3. The tool of claim 2 or claim 3, wherein the inner element (202) includes a more proximal reduced diameter (218, 219) and a more distal reduced diameter (220), the more proximal reduced diameter (218, 219) is configured to translate the inner element (202) distally and the more distal reduced diameter (220) is configured to facilitate passive disengagement of the threaded distal end (208) from the screw (100).

4. The tool of any one of claims 1-3, wherein the button (210) includes a spring-loaded button.

5. The tool of any one of claims 2-4, further comprising an inner knob (226) on the inner element (202) including the button (210) and a locking button (300, 600, 700, 800, 900, 1000, 1100, 1200, 1300, 1400, 1500, 1600, 1700, 1800, 1900, 2000, 2100, 2200).

6. The tool of claim 5, further comprising an angled slot (230) on the outer knob (212) configured to attach to the inner knob (226) and allow axial translation from the first position (214) to the second position (216).

7. The tool of claim 5 or claim 6, wherein the locking button (300, 600, 700, 800, 900, 1000, 1100, 1200, 1300, 1400, 1500, 1600, 1700, 1800, 1900, 2000, 2100, 2200) is configured to translate inward via an interface between a ramped inner surface of the outer knob (212) and the outer knob (212), when the outer knob (212) is moved distally into the second position (216).

8. The tool of any one of claims 5-7, further comprising a cut spline (228, 628, 728, 828, 928, 1028, 1328, 2228) on the inner element (202) that engages with the locking button (300, 600, 700, 800, 900, 1000, 1100, 1200, 1300, 1400, 1500, 1600, 1700, 1800, 1900, 2000, 2100, 2200) in the second position (216).

9. The tool of any one of claims 5-8, wherein the locking button (300, 600, 700, 800) includes a pawl (636, 738, 838) configured to engage the cut spline (628, 728, 828) on the inner element (602, 702, 802) to prevent rotation in one or both directions.

10. The tool of any one of claims 5-9, wherein the locking button (300, 900, 1000, 1100, 1400, 1500, 1600, 1700, 1800, 1900, 2000, 2100, 2200) includes one or more teeth (937, 1037, 1137, 1437, 1537, 1637, 1737, 1837, 1937, 2037, 2137, 2237) configured to engage the cut spline (228, 928, 1028, 2228) on the inner element (202, 902, 1002, 2202) to prevent rotation in one or both directions.

11. The tool of any one of claims 5-10, wherein the locking button (900, 1000) includes a cantilever beam flexure (944, 1044) configured to engage the cut spline (902, 1002) and prevent rotation in one direction.

12. The tool of any one of claims 5-11, wherein the locking button (1300) includes a cartwheel flexure (1346), with a pivot point (1341) at an intersection of two connected flexures, configured to engage the cut spline (1302) and prevent rotation in one direction.

13. The tool of any one of claims 5-12, wherein the locking button (1100) includes a small-length flexural pivot (1154) configured to engage the cut spline and prevent rotation in one direction.

14. The tool of any one of claims 5-13, wherein the locking button (1200) includes a cross-axis flexural pivot (1248), with a pivot point (1241) at an intersection of two disconnected flexures, configured to engage the cut spline and prevent rotation in one direction.

15. The tool of any one of claims 5-14, wherein the locking button (1400, 1500, 1600, 1700, 1800, 1900, 2000, 2100) includes a beam flexure (1450, 1550, 1650, 1756a, 1756b, 1856a, 1856b, 1956a, 1956b, 2050, 2150) with teeth (1437, 1537, 1637, 1737, 1837, 1937, 2037, 2137) configured to engage the cut spline and prevent rotation in one or both directions.
